Europäisches Patentamt

European Patent Office

Office européen des brevets

(11) Veröffentlichungsnummer: **0 184 643**
A2

(12) **EUROPÄISCHE PATENTANMELDUNG**

(21) Anmeldenummer: 85113353.8

(22) Anmeldetag: 22.10.85

(51) Int. Cl.⁴: **A 61 B 17/36,** A 61 N 5/06,
G 02 B 6/00

(30) Priorität: 08.12.84 DE 3444824

(43) Veröffentlichungstag der Anmeldung: **18.06.86**
**Patentblatt 86/25**

(84) Benannte Vertragsstaaten: **AT BE CH DE FR GB IT LI LU
NL SE**

(71) Anmelder: **Messerschmitt-Bölkow-Blohm Gesellschaft
mit beschränkter Haftung, Robert-Koch-Strasse,
D-8012 Ottobrunn (DE)**

(72) Erfinder: **Wondrazek, Fritz, Dr. Dipl.-Phys.,
Riegelstrasse 27, D-8068 Pfaffenhofen (DE)**
Erfinder: **Hahn, Andreas, Dipl.-Ing. (FH),
Ringbergstrasse 19, D-8029 Sauerlach (DE)**
Erfinder: **Einars, Wolfram, Dr. Dipl.-Ing.,
Dianastrasse 13, D-8011 Neukeferloh (DE)**

(54) Einrichtung für intrakorporale Laserbestrahlung.

(57) Die Erfindung betrifft eine Einrichtung, bestehend aus
einem Laser (1) und einem Lichtleiter (2) für intrakorporale
Laserbestrahlung, insbesondere zur Abtragung von Gewebe oder von Ab- bzw. Auflagerungen an Gewebe, bei der
der Lichtleiter und das im wesentlichen rein mechanische
Einkoppelstück (2.1) entweder nach jedem Gebrauch leicht
zu sterilisieren oder so billig herzustellen sind, daß sie als
Einweginstrument wirtschaftlich verwendet werden können.

EP 0 184 643 A2

0184643

07.12.84, 0318A
Mn/bk
9660

MBB

Patentabteilung

Einrichtung für intravaskuläre Laserbestrahlung

Die Erfindung betrifft eine Einrichtung für intrakorporale Laserbestrahlung, insbesondere zur Abtragung von Gewebe oder von Ab- bzw. Auflagerungen an Gewebe.

Die bisher für medizinische Zwecke verwendeten Einrichtungen zur Laserbestrahlung bestehen aus einem Lasergerät geeigneter Leistung und Wellenlänge, an welches eine Lichtleitfaser über eine spezielle Einkopplung mit optischen Elementen angeschlossen wird(z.B. DE-OS 31 21 287). Dabei bildet das Einkoppelstück mit einer Einkoppeloptik zusammen mit der Lichtleitfaser eine Einheit, welche in den parallelen Strahlengang des Lasers mittels eines geeigneten Anschlusses eingeschoben wird. Ein derartiger Lichtleiter ist relativ teuer und muß nach jeder Behandlung sterilisiert werden, wodurch die Gefahr einer Beschädigung der optischen Komponenten besteht. Nachteilig ist weiterhin, daß die Lichtleitfaser im Körper des Patienten mit Röntgenkameras nur unzureichend sichtbar ist. Ein weiterer Nachteil besteht darin, daß durch die vergleichsweise hohe übertragene Laserleistung das austrittsseitige Ende der Lichtleitfaser durch verdampfendes Gewebe verdreckt und nach wenigen Behandlungen optisch nachbearbeitet werden muß.

Aufgabe der Erfindung ist es, eine Einrichtung für intrakorporale Laserbestrahlung zu schaffen, welche die obengenannten Nachteile nicht mehr aufweist, bzw. deren Folgen erträglicher macht. Die Lösung dieser Aufgabe gelingt durch eine nach den kennzeichnenden Merkmalen des Patentanspruchs 1 ausgebildete Einrichtung.

0184643

07.12.84, 0318A
Mn/bk
9660

Die Kombination der die Erfindung kennzeichnenden Merkmale ermöglicht es, den Lichtleiter und das im wesentlichen rein mechanische Einkoppelstück entweder nach jedem Gebrauch leicht zu sterilisieren oder so billig herzustellen, daß sie als Einweginstrument wirtschaft- lich verwendet werden können.

Die weiteren Vorteile der Erfindung sowie die vorteilhaften Weiterbildungen sollen im folgenden anhand eines teilweise schematisch dargestellten Ausführungsbeispieles beschrieben werden.

Es zeigen:

Fig. 1 die Verbindungsstelle zwischen einem Laser und einem Lichtleiter einer Einrichtung für intrakorpolare Laserbestrahlung;

Fig. 2 das austrittsseitige Ende der Lichtleitfaser.

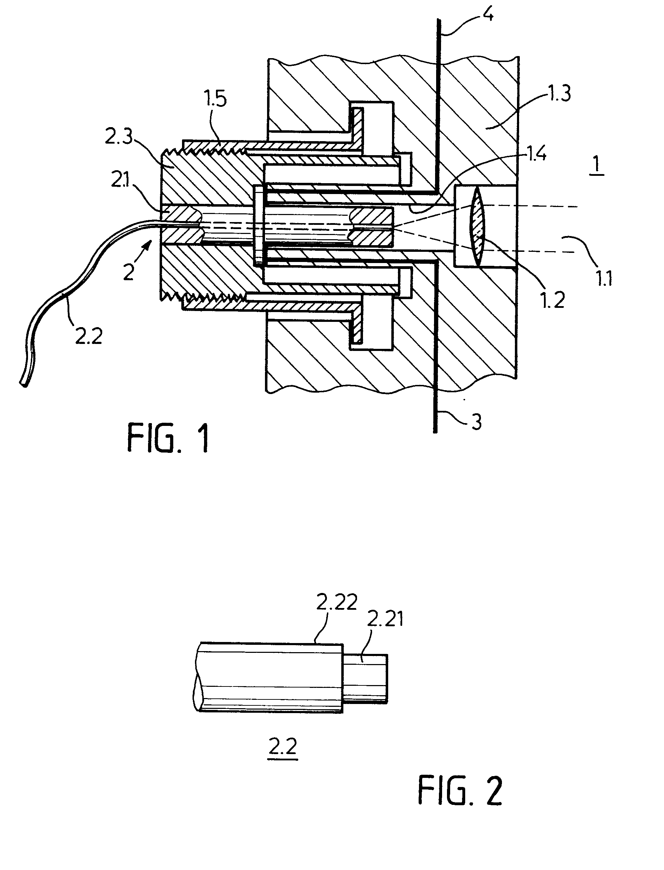

Fig. 1 zeigt ein nicht näher dargestelltes Lasergerät 1, dessen parallele Strahlung 1.1 mittels einer Einkoppeloptik 1.2 innerhalb der Austrittsöffnung eines Anschlusses 1.3 auf das eintrittsseitige Ende eines Lichtleiters 2 fokussiert wird. Die Wellenlänge der Laserstrahlung sollte für die intrakorpolare Anwendung > 0,32 µ sein. Der Anschluß 1.3 weist eine präzise gearbeitete Bohrung 1.4 auf, welche als Führung für ein zylindrisches Einkoppelstück 2.1 dient. In einer zentralen Bohrung des Einkoppelstückes 2.1 ist die Lichtleitfaser 2.2 hindurchgeführt und darin befestigt. Das Einkoppelstück 2.1 ist mit einer, das Lichtleiterende konzentrisch umgebenden Hülse 2.3 befestigt, welche am äußeren Umfang ein Gewinde aufweist. In dieses Gewinde greift eine in dem Anschluß 1.3 verankerte Überwurfmutter 1.5 ein, mittels welcher das Einkoppelstück 2.3

0184643

07.12.84, 0318A
Mn/bk
9660

**MBB**

Patentabteilung

3

bis zu einem definierten Anschlag in die Bohrung 1.4 hineingezogen wird. Die Optik 1.2 ist so dimensioniert, daß in dieser Position des Einkoppelstückes eine optimale Einkopplung der Laserstrahlung in die Lichtleitfaser stattfindet. Diese Position wird zusätzlich noch dadurch überwacht, daß mittels des definierten Anschlages des Einkoppelstückes 2.1 zwei elektrische Kontakte 3 und 4, welche isoliert durch den Anschluß 1.3 geführt sind, kurzgeschlossen werden.

Fig. 2 zeigt das Ende der Lichtleitfaser 2.2, welche in Fig. 1 zur besseren Darstellung wesentlich dicker eingezeichnet ist als sie im Verhältnis zu den übrigen Bauteilen ist. Die Lichtleitfaser ist als Stufenindexfaser ausgebildet und hat bei einer zu übertragenden Laserleistung von 50 W typischerweise einen Kerndurchmesser von 200 μ und einen Manteldurchmesser von 240 μ. Die numerische Apertur sollte > 0,2 sein und der minimale Biegeradius < 1 cm. Die eigentliche Lichtleitfaser 2.21 ist von einer weiteren Mantelschicht (Coating) 2.22 umgeben, welche gewebeverträglich und beständig gegen Kochsalzlösung ist und zur Durchführung in einem Katheter eine geringe Reibung aufweist. Zur Erkennung der Lichtleitfaser im Körper unter Röntgenstrahlung kann die Mantelschicht 2.22 am Ende der Lichtleitfaser einen zusätzlichen Metallring aufweisen, bzw. metallisch beschichtet seinoder einen in der Masse der Mantelschicht fein dispergierten, Röntgenstrahlung absorbierenden bzw. reflektierenden Stoff beinhalten.

Die Lichtaustrittsseite der Lichtleitfaser 2.21 ist von guter optischer Qualität, was sich bei einer Stufenindexfaser durch Brechen oder Polieren erreichen läßt. Von ähnlich guter optischer Qualität muß auch die Lichteintrittsseite sein; bei einer Faserlänge von ty

*MBB*

Patentabteilung

07.12.84, 0318A
Mn/bk
9660

pischerweise 3 m sollte auch die Dämpfung der Lichtleitfaser gering sein, z.B. bei einer Wellenlänge von ca. 1 µ kleiner als 5 dB/km.

Zur Vermeidung von Beschädigungen der Mantelschicht 2.22 am austrittseitigen Ende der Lichtleitfaser durch rückgestreute Laserstrahlung ist es zweckmäßig, das Ende der Lichtleitfaser in einem Bereich von etwa 2 mm von der Mantelschicht zu befreien.

**MBB**

Patentabteilung

1

07.12.84, 0318A
Mn/bk
9660

018 4643

Einrichtung für intrakorporale Laserbestrahlung

Patentansprüche

1. Einrichtung für intrakorporale Laserbestrahlung, insbesondere zur Abtragung von Gewebe oder von Ab- bzw. Auflagerungen an Gewebe g e k e n n z e i c h n e t durch die Kombination folgender Merkmale:

a) ein Laser (1) mit ausgangsseitigem Anschluß zur lösbaren Verbindung mit einem Einkoppelstück (2.1) einer Lichtleitfaser (2.2), a.a) wobei die Laserleistung mindestens 1 W beträgt und

a.b) wobei der Anschluß eine mit diesem fest verbundene Einkoppeloptik (1.2) aufweist und

b) ein Lichtleiter (2) mit

b.a) einem eintrittsseitigen Einkoppelstück (2.1) zur mechanischen Verbindung mit und exakter Führung in dem Anschluß (1.3) des Lasers (1),

b.a.a) wobei das Einkoppelstück (2.1) keine optischen Einkoppelelemente aufweist,

b.b) einer mit dem Einkoppelstück (2.1) fest verbundenen Lichtleitfaser (2.2) mit ein- und/oder austrittsseitigen gebrochenem oder poliertem Ende und mit

b.c) einem zumindest in der Nähe des austrittsseitigen Endes der Lichtfaser (2.2) angeordneten Überzug aus einem Röntgenstrahlung reflektierenden bzw. absorbierenden Material.

2. Einrichtung nach Anspruch 1, dadurch g e k e n n z e i c h n e t , daß die Lichtleitfaser einen minimalen Biegeradius kleiner 1 cm aufweist.

0184643

MBB
Patentabteilung

07.12.84, 0318A
Mn/bk
9660

3. Einrichtung nach Anspruch 1 oder 2, dadurch g e k e n n z e i c h n e t , daß die Lichtleitfaser (2.2) eine Stufenindexfaser ist.

4. Einrichtung nach einem der Ansprüche 1 bis 3, dadurch g e k e n n z e i c h n e t , daß das austrittsseitige Ende der Lichtleitfaser (2.2) in einem Bereich von 1 - 4 mm von der Mantelschicht (Coating) (2.22) befreit ist.

5. Einrichtung nach einem der Ansprüche 1 bis 4, dadurch g e k e n n z e i c h n e t , daß die Lichtleitfaser (2.2) zusammen mit dem Einkoppelstück (2.1, 2.3) eine sterile und pyrogenfreie, verpackbare Einheit bildet.

6. Einrichtung nach einem der Ansprüche 1 bis 5, dadurch g e k e n n z e i c h n e t , daß die Laserstrahlung (1.1) eine Wellenlänge > 0,3 $\mu$ aufweist.

7. Einrichtung nach einem der Ansprüche 1 bis 6, dadurch g e k e n n z e i c h n e t , daß der Anschluß eine elektrische Kontrolleinrichtung (3,4) aufweist, welche bei nicht vollständig eingestecktem Einkoppelstück (2.1) der Lichtleitfaser (2.2) ein elektrisches Signal liefert.

FIG. 1

2.2

FIG. 2